# EUROPEAN PATENT SPECIFICATION

(11) **EP 2 094 295 B1**
(45) Date of publication and mention of the grant of the patent: **22.06.2011**
(21) Application number: 07866799.5
(22) Date of filing: 13.12.2007
(51) Int. Cl.: A61K 39/00, G01N 33/50

(54) **METHOD FOR THE DIAGNOSIS AND/OR MONITORING OF INVASIVE ASPERGILLOSIS**
VERFAHREN ZUR DIAGNOSE UND/ODER ÜBERWACHUNG VON INVASIVER ASPERGILLOSE
MÉTHODE DE DIAGNOSTIC ET/OU DE SURVEILLANCE DE L'ASPERGILLOSE INVASIVE

(30) Priority: 19.12.2006 IT MI20062448
(43) Date of publication of application: 02.09.2009
(73) Proprietor: Universita' Degli Studi Di Modena E Reggio Emilia, 41100 Modena (IT)
(72) Inventor: LUPPI, Mario, 41015 Nonantola Modena (IT); BAROZZI, Patrizia, 41100 Modena (IT); POTENZA, Leonardo, 41100 Modena (IT); TORELLI, Giuseppe, 41100 Modena (IT)
(74) Representative: Pistolesi, Roberto
(86) International application number: PCT/IT2007/000867
(87) International publication number: WO 2008/075395

(56) References cited:
- WO-A-03/018051
- WO-A-2005/107791
- US-A1- 2002 061 544
- TRULL A K ET AL: "IMMUNOGLOBULIN G ELISA FOR DIAGNOSIS OF INVASIVE ASPERGILLOSIS RETROSPECTIVE STUDY OVER 15 YEARS OF TRANSPLANT RECIPIENTS" JOURNAL OF CLINICAL PATHOLOGY (LONDON), vol. 38, no. 9, 1985, pages 1045-1051, XP002478378 ISSN: 0021-9746
- GAZIANO R ET AL: "Anti-Aspergillus fumigatus efficacy of pentraxin 3 alone and in combination with antifungals" ANTIMICROBIAL AGENTS AND CHEMOTHERAPY, AMERICAN SOCIETY FOR MICROBIOLOGY, WASHINGTON, DC, US, vol. 48, no. 11, November 2004 (2004-11), pages 4414-4421, XP002337599 ISSN: 0066-4804
- BOZZA SILVIA ET AL: "A dendritic cell vaccine against invasive aspergillosis in allogeneic hematopoietic transplantation." BLOOD, vol. 102, no. 10, 15 November 2003 (2003-11-15), pages 3807-3814, XP002478379 ISSN: 0006-4971
- RAMADAN G ET AL: "Generation of Th1 T cell responses directed to a HLA Class II restricted epitope from the Aspergillus f16 allergen." CLINICAL AND EXPERIMENTAL IMMUNOLOGY FEB 2005, vol. 139, no. 2, February 2005 (2005-02), pages 257-267, XP002488942 ISSN: 0009-9104
- RAMADAN G ET AL: "Generation of cytotoxic T cell responses directed to human leucocyte antigen Class I restricted epitopes from the Aspergillus f16 allergen." CLINICAL AND EXPERIMENTAL IMMUNOLOGY APR 2005, vol. 140, no. 1, April 2005 (2005-04), pages 81-91, XP002488943 ISSN: 0009-9104

## Description

### SUMMARY DESCRIPTION OF THE INVENTION

The present invention is a method for the diagnosis of invasive aspergillosis which comprises carrying out an in vitro immunoenzymatic ELISPOT assay wherein the biological fluid drawn from the patient is placed in contact with an antigen of *Aspergillus fumigatus,* which is characterized in that it comprises verifying the presence of IFN-γ producer T cells and IL-10 producer T cells and/or the ratio between IFN-γ producer T cells and IL-10 producer T cells.

In the method according to the present invention, said antigen is preferably an extractive antigen of *Aspergillus fumigatus;* more preferably, said antigen is obtained from conidia or hyphae of *Aspergillus fumigatus.*

According to an embodiment of the invention, said antigen is obtained through the following passages:
- incubation of the conidia for 3 days,
- collection of the conidia by means of washing of the fungal culture with sterilised water,
- filtration and subsequent centrifugation of the washing liquid,
- elimination of the supernatant,
- freezing and subsequent unfreezing of the conidia pellet,
- suspension in sterilised water,
- boiling and subsequent centrifugation,
- possible freezing.

According to another embodiment of the invention, said antigen is obtained through the following passages:
- collection of hyphae from culture plates though scraping and subsequent suspension in sterilised water,
- filtration and subsequent centrifugation,
- elimination of the supernatant,
- freezing and subsequent unfreezing of the mycete pellet,
- homogenisation of the mycete in the presence of a volume of inert material beads (preferably glass beads) equal to the volume of the mycete,
- centrifugation,
- possible freezing.

According to an embodiment of the invention, said biological fluid is selected from among blood, bronchoalveolar lavage fluid or pleural fluid.

According to another embodiment of the invention, said biological fluid is selected from among all the mononuclear cells of the blood, of the bronchoalveolar lavage fluid, of the pleural fluid, or T lymphocytes of the blood, of the bronchoalveolar lavage fluid, of the pleural fluid.

In the method according to the present invention, the threshold value for IFN-γ or IL-10 is preferably in the range of 15 - 25 SFCs; more preferably, said threshold value is 20 SFCs.

### PRIOR ART

Invasive fungal infections (IFI) represent an important cause of morbidity and mortality in patients affected by malignant hemopathies, not only subjected to allogeneic marrow transplants but also standard chemotherapy. The risk of death of patients affected by IFI even attains percentages of 90%.

The responsible etiological agent of the IFIs is represented by fungi of the species *Aspergillus,* which accounts for nearly 90% of all invasive infections. The causes of such a high mortality lie in the lack of diagnostic methods which permit a diagnosis of Invasive Aspergillosis (IA). The instruments available to the clinical haematologist are in fact limited by invasiveness, slowness, low sensitivity, lack of standardisation and variable kinetics (Hope WW 2005; Mennink-Kersten M 2004; Patterson TF 2005).

The methods currently in use for the diagnosis of invasive aspergillosis are the histological exam, the high-resolution computed axial tomography of the thorax (HRCT), culture exams, serologic exams (galactomannan, 1-3-beta-glucan) and the polymerase chain reaction for fungus nucleic acids (PCR).

Only the histological exam and the culture exam, however, give absolute confirmation of IA, but have invasiveness limits and low sensitivity (positive in a percentage less than about 30%). HRCT is not specific, while GM has an extremely variable sensitivity (in a range between 30% - 100%) since it is influenced by numerous factors, which limit its application, including the use of the antifungal prophylaxis and/or the patients' diet. Both the beta-glucan and the PCR still lack standardisation and have completely unpredictable kinetics. Moreover, the detection of the *Aspergillus* DNA through PCR is prevented by the difficulties of comprehension of the release and kinetics of the fungus DNA, in addition to technical barriers (Hope WW, Denning DW. Laboratory diagnosis of invasive aspergillosis. Lancet Infect Dis 2005; 5: 609-622).

Recent studies, first in rodents, then also in humans, demonstrated that the adaptive immunity, or that represented by the T cells, carries out a predominant role in the defence of the host against fungi (Cenci E 1997; Hebart H 2002; Romani L 2004). In particular, a cell-mediated immunity polarised with type 1 T helper cells (TH1), producer of gamma-interferon (IFN-γ) is protected against fungal infections, especially IA; while a cell-mediated immunity polarised with type 2 T helper cells (TH2), producer of interleukin 10 (IL-10), is permissive with regard to IA (Cenci E 1997; Hebart H 2002; Romani L 2004).

The ELISPOT method (*ElisaSpot or Enzyme-linked ImmunoSpot Assay*) has shown to be a sensitive and specific instrument in the diagnosis of microbacterial infection of tuberculosis or in infections of viral origin, both in immunocompetent subjects and in immunodepressed subjects (Lalvani 2001), through the research of microbacterium-specific T cells, or for the isolation of T cells specific for the Epstein-Barr virus in kidney transplant patients (Comoli 2002).

ELISPOT is an assay based on the cellular immunological response towards a specific antigen. Such method is generally carried out on 96 well plates coated with specific monoclonal antibodies in which the cells and the specific antigen are incubated for 6-48 hours.

During such period, the cell response occurs and the specific cytokines are produced, according to the type of antibody/antigen used, which generate spots which are detected and counted through common image analysis software.

The ELISPOT method, for a generic evaluation of the blood cell activity, is described in the European patent application EP0957359 (Volkmar et al.), incorporated here for reference purposes.

The ELISPOT method applied to the T lymphocytes for the diagnosis or monitoring of diseases such as hepatitis B, hepatitis C, tuberculosis, malaria, HIV or influenza is described in the international patent application WO98/23960 (Lalvani et al.), incorporated here for reference purposes.

The ELISPOT method applied to the T lymphocytes reactive to the antigens deriving from the protein ESAT-6, expressed by *Mycobacterium Tubercolosis,* for the diagnosis of tuberculosis or even only at the contact of the host with the pathogen (latent disease), is described in the international patent application WO00/26248 (Lalvani et al.), incorporated here for reference purposes.

A further ELISPOT method applied to the T lymphocytes reactive not only to the antigens deriving from the ESAT-6 but also from the protein CFP-10 is described in the international patent application WO2004/005925 (Lalvani et al.), incorporated here for reference purposes.

The ELISPOT method, therefore, was utilised up to now for infections and diseases of viral origin, or for *Mycobacterium Tuberculosis.*

Methods for the diagnosis of invasive aspergillosis by means of immunoenzymatic assays with an antigen of *Aspergillus fumigatus* are disclosed by Trull et al., Journal of Clinical Pathology, Vol. 38, n. 9, 1985, pages. 1045-1047 and by US 2002/061544.

In Gaziano et al., Antimicrobical Agents and Chemotherapy, Vol. 48, n. 11, 2004, pages. 4414-4421, the ELISPOT assay has been used to enumerate the number of splenocytes producing IFN-g induced by a single infusion of Pentraxin 3 (PTX-3) in transplanted mice either before or after the infection with *Aspergillus fumigatus.*

Bozza et al., Blood, Vol. 102, n. 10, 2003, pages. 3807-3814, describes a dendritic cell vaccine against invasive aspergillosis in allogenic hamatopoietic transplantation.

In Ramadan et al., Clinical and Experimental Immunology, Vol. 139, February 2005, pages 257-267, ELISPOT has been used to detect the frequency of peripheral blood mononuclear cells able to respond to *Aspergillus* overlapping peptides deriving from the Aspf16 protein in healthy donors, to understand factors determining the specific immune response against *Aspergillus* (pag. 265 column 2, second paragraph).

In Ramadan et al., Clinical and Experimental Immunology, Vol. 140, April 2005, pages 81-91, ELISPOT has been used to detect the presence of either CD4-positive or CD8-positive T cells, specific for HLA class I restricted epitopes of the previously mentioned Aspf16 protein in healthy donors.

WO03/018051 reports about an orally available fungal vaccine. The ELISPOT assay is cited as a further method to test the effect of the immunization with the vaccine.

In WO2005/107791, ELISPOT is used to detect mice splenocytes producing IFN-g after the infusion of PTX-3.

### DETAILED DESCRIPTION OF THE INVENTION

It has now been found that immunoenzymatic assays, in particular ELISPOT, can be advantageously used for the diagnosis, in particular an early diagnosis of fungal infections, in particular in the case of invasive aspergillosis.

The object of the invention is in fact that of using such assays, in particular the ELISPOT assay for identifying and counting Aspergillus-specific T cells, for the simple detection or to determine a quantitative ratio between the IFN-γ producers and the IL-10 producers, in order to define the infection risk, for the diagnosis of invasive aspergillosis.

The object of the present invention is therefore a method for the diagnosis of the invasive aspergillosis which comprises carrying out an *in vitro* immunoenzymatic assay on microtitration plates, such as for example of 96 well type, characterised in that it places the biological fluid drawn from the patient in contact with an antigen of *Aspergillus fumigatus,* in particular an extractive antigen *Aspergillus fumigatus.*

According to the present invention, said method includes the identification and count of Aspergillus-specific T cells, in order to verify the presence and/or define a ratio between the IFN-γ producers and the IL-10 producers.

The threshold value for IFN-γ or IL-10 which permits defining the positive result of the method according to the present invention is in the range of 15 - 25 SFCs (*Spot Forming Cells*), preferably 20 SFCs.

According to the present invention, said immunoenzymatic assay is ELISPOT (ElisaSpot or Enzyme-linked ImmunoSpot Assay) and said antigen, preferably extractive antigen, can be obtained from conidia of *Aspergillus fumigatus* or from hyphae of *Aspergillus fumigatus.*

The conidia (or conidiospores) are predominantly unicellular reproductive structures which are formed at the top of the of hyphae that, in turn, are unicellular or pluricellular filaments of cylindrical form, that arranged on top of each other forma, form the mycelium or the vegetative body of the fungi.

In particular, according to an embodiment of the present invention, said antigen is obtained from conidia of *Aspergillus fumigatus* which is collected after three days of culture, preferably Sabouraud agar medium, filtered through sterilised gauze, and finally killed with heat, for 1 hour at 100°C. Afterwards, they are washed in a saline solution (PBS) and preserved at -20°C.

According to a further embodiment of the present invention, said antigen, preferably extractive antigen, is obtained from hyphae of *Aspergillus fumigatus* which is homogenised, for example by using a mortar with beads of an inert material, preferable glass beads in a buffer, preferably 50 mM Tris-Hcl with a pH of about 7.5. The protein extract thus obtained is recovered after centrifugation between 10 and 20000 rpm for about 10 minutes and preserved at a temperature in the range of about -30 to -10°C, preferably -20°C.

For the purposes of the present invention, with the expression "antigen of *Aspergillus fumigatus*" it is therefore intended constituents of the fungal structure treated in a manner such that they are capable of eliciting a response of specific T cells.

The biological fluid used according to the method of the present invention can be blood, bronchoalveolar lavage fluid (BAL) or pleural fluid from patients at risk of IA or already infected.

In particular, such fluid can be obtained from an intravenous citrate sample, preferably 20 ml, from pleural fluid citrate, preferably 20 ml, or from a citrated BAL fluid sample, preferably 30 ml.

Said blood can be used according to the method of the present invention as total blood or as isolated T lymphocytes.

According to the present invention, the sample of blood, pleural fluid or bronchoalveolar lavage fluid is diluted with an equivalent volume of culture medium, for example RPMI 1640, and is then subjected to density gradient centrifugation (Ficoll, Lymphoprep) in order to separate the population of the mononuclear cells (PBMCs). After 2 washings, for example in RPMI 1640, the cells are frozen in liquid nitrogen, preferably in the presence of 20% DMSO and 50% foetal bovine serum (FBS) for the ELISPOT experiments.

The cell samples are then slowly unfrozen in a 35-40°C thermostatic bath, preferably at 37°C. DNAse is added to the final concentration of about 10mg/ml, and it is left to operate at room temperature for about 20 minutes. The cells are transferred into a test tube and delicately resuspended with about 10 ml of RPMI 1640 medium added with about 5% FBS which is added drop by drop. After a first centrifugation at about 1000 rpm for about 10', the cellular pellet obtained is once again resuspended in a R10 culture medium (RPMI 1640; 10% FBS; 1% sodium pyruvate; 1% ampicillin; 0.5% gentamicin; 18 UI/ml IL-2) and then the cells are counted in a Burker chamber.

For the separation of the CD3+ T cells, the "Pan T cell Isolation Kit human" distributed by Miltenyi Biotech S.r.l. (Calderara di Reno, Bologna, Italy) was used. The separation of the CD3+ T cells occurs by depletion of the non-T cells (negative selection). The non-T cells marked with a mixture of biotin-conjugated monoclonal antibodies (CD14, 16, 19, 36, 56, 123 and Glycophorin A), are made to react with anti-biotin monoclonal antibodies conjugated with magnetic microbeads. The passage of the marked cells (non-T cells) and non-marled cells (CD3+ T cells) through a MACS-MS column (Miltenyi) in the presence of a magnetic field determines the passage of the CD3+ T cells and the blocking of the non-T cells. The separation procedure was carried out on cell samples after unfreezing, following the indications described by Miltenyi.

According to a further embodiment of the present invention, said antigen can be obtained from conidia of *Aspergillus fumigatus* which are collected after 2-4 days of culture (for example Sabouraud agar medium) through washing of the surface layer of the fungal culture with sterilised water. The washing liquid obtained is filtered through sterilised gauze and subjected to centrifugation between 10000 - 15000 rpm, preferably at 12000 rpm for 5-15 minutes, preferably 10 minutes. The supernatant is eliminated and the pellet of conidia thus obtained is frozen in liquid nitrogen for about 12 hours. Afterwards, the conidia pellet is unfrozen, resuspended in sterilised water and made to boil in a water bath for about 1 hour and 30 minutes. After centrifugation (about 10 minutes at 12000 rpm) in order to eliminate the supernatant, the pellet of conidia is resuspended in about 500 microlitres of PBS 1X. The conidia thus obtained are used in the Elispot experiment at the concentration of about 100,000 conidia/ml.

The obtained antigen can be used immediately or it can be stored by means of freezing at about -20°C and used later.

According to a further embodiment of the present invention, said extractive antigen can be obtained from hyphae of *Aspergillus fumigatus* which are collected by scraping several culture columns with a scalpel blade and resuspended in sterilised water. After having vortexed, one proceeds with the filtration through sterilised gauze for eliminating the possible agar. The mycete thus obtained is subjected to centrifugation at about 15000 rpm per circa 10 minutes, then the supernatant eliminated, it is frozen in liquid nitrogen for about 12 hours. Afterwards, the unfrozen mycete is homogenised in a mortar in the presence of a volume of inert material beads, preferably glass beads, volume equal to that of the mycete, and in a buffer, preferably 50 mM Tris-Hcl buffer, at a pH of about 7.5. The protein extract thus obtained is recovered after centrifugation at about 14000 rpm for about 20'. After the evaluation of the protein content, the extract from hyphae, is used in the Elispot experiment at the concentration of about 6-10 micrograms/ml.

The obtained antigen can be used immediately or it can be stored through freezing at about -20°C and used later.

### EXPERIMENTAL PART

1. ELISPOT method for Aspergillus-specific T cell producers of gamma-IFN-For all ELISPOT experiments according to the present invention, 96 well microtitration plates distributed by Mabtech (Nacka Strand, Sweden) were used. The bottom of the wells of the plate, composed of nitrocellulose, is coated with a monoclonal antibody specific for IFN-γ. The plates were removed from the packaging and washed 4 times with PBS 1X (200 µl/well). The plates were fixed with R10 (200 µl/well), and incubated at room temperature for ≥30'. The fixing means are removed and 100,000-200,000 cells of medium R10 are dispensed in the single wells (final volume of 100-150 µlwell) and then stimulated with conidia inactivated by the heat (1x105 conidia/ml) or with protein extract (6-10 µg/ml) during an incubation at 37°C in the presence of CO2 (5%) for about 20 hours.

The positive controls consisted of the same cells incubated with phytohemagglutinin (PHA) (25 µg/ml) or with a pool of 23 viral peptides, restricted for the greater histocompatibility complex class I, deriving from the cytomegalovirus, Epstein-Barr virus and from the influenza virus (CEF Peptide pool, Mabtech); the negative control consisted of the single cells, without any stimulation. All experiments were conducted in triplicate.

After incubation, the plate is emptied and subjected to 5 successive washings with 1x PBS and 0.5% Tween 20. The cytokine-antibody complexes are coloured by means of enzymatic reaction and addition of a chromogenic substrate: in every single well, 50 µl are distributed of secondary antibody conjugated with alkaline phosphatase, distributed by Mabtech, diluted 1:200 in 1x PBS and after 90' of incubation at room temperature the substrate (BCIP/NTB-plus) is added, it too distributed Mabtech. The reaction with the substrate is stopped after 10' by washing the plate with running water. The nitrocellulose membrane is dried out in contact with air for at least 4 h. Every single spot which appears on the bottom of the well corresponds with the cytokine secretion of a single cell (spot forming cell: SFC). The spots are automatically counted by means of an image analysis instrument (AID ELISPOT Reader System, Amplimedical) managed by a software capable of providing an easy and quick evaluation of the spots based on their size and intensity.

### 2 ELISPOT method for Aspergillus-specific T cell producers of IL-10

For all ELISPOT experiments, 96 well microtitration plates were used (Multiscreen HTS IP Sterile Plate) distributed by Millipore (Bedford, MA, USA). In this case, the plates are not previously coated, but require coating with the anti-IL-10 monoclonal antibody. One proceeds in the following manner: the plates are removed from the packing and the membrane is soaked with 70% Ethanol (15 (µl/well) for one minute at room temperature. The plates are then washed 5 times with distilled water (200 µl/well). The antibody anti-IL-10 (9D7), distributed by Mabtech, used for coating, is diluted to 15 µg/ml in sterile 1x PBS and pH 7.4 and is added in 100 µl quantities per well to the plate which is incubated for a night at +4-8°C.

After incubation, the plate is subjected to successive washings with 1x PBS (200 µl/well). The wells are fixed with a medium containing the same 10% FBS used for suspending the cells (200 µl/well), and incubated at room temperature for ≥30'. The fixing means are removed and 50,000-100,000 cells in R10 are dispensed in the single wells (final volume of 100-150 µl/well) and then stimulated with conidia inactivated by the heat (1x105 conidia/ml) or with protein extract (6-10 µg/ml) during incubation at 37°C in the presence of (5%) CO2 for about 20 hours. The positive controls are composed by the same cells incubated with phytohemagglutinin (PHA) (25 µg/ml) or with anti-CD3 (Mabtech); the negative control consisted of the single cells, without any stimulation. All experiments were carried out in triplicate.

After incubation, the plate is emptied and subjected to 5 successive washings with 1x PBS. The secondary antibody (12G8-biotin) is diluted to 1 µg/ml in PBS-0.5% FCS, added to the quantity of 100 µl/well and incubated for 2 h at room temperature. One proceeds with 5 successive washings with 1x PBS. The Streptavidin-alkaline phosphatase complex (1:1000) is diluted in PBS-0.5% FCS, added to the quantity of 100 µl/well and incubated for 1 h at room temperature. One proceeds with 5 successive washings with 1x PBS. The solution containing the substrate (BCIP/NBT) is added in quantity of 100 µl/well and left to develop until the spots appear. The colour development is blocked with abundant washing in running water. The basal membrane of the plate is removed and also the base of the plate is washed. The nitrocellulose membrane is dried in contact with the air for at least 4 h. The spots are then automatically counted by means of an image analysis instrument (AID ELISPOT Reader System, Amplimedical) managed by a software capable of providing a quick and easy evaluation of the spots based on their size and intensity.

### EXAMPLES

### Example 1

### Preparation of the antigen from conidia of Aspergillus fumigatus

After three days of culture of the *Aspergillus fumigatus* Sabouraud agar medium, the conidia are collected by means of washing of the surface layer of the culture with 30 ml of sterilised water.

The washing liquid is filtered through sterilised gauze and is subsequently subjected to centrifugation at 12000 rpm for 10 minutes.

The supernatant is eliminated and one proceeds with the freezing of the conidia pellet for 12 hours in liquid nitrogen. The conidia pellet is then unfrozen and suspended in 1-2 ml of sterilised water and boiled in a water bath for 1.5 hours. One then proceeds with a further centrifugation at 12000 rpm for 10 minutes and the conidia pellet is resuspended in 500 µl of 1x PBS.

Finally, the obtained conidia can be directly used in the ELISPOT experiment according to the present invention at a concentration of 100,000 conidia/ml. Alternatively, the conidia are frozen at -20°C until the time of use, according to the present invention.

### Example 2

### Preparation of the antigen from hyphae of Aspergillus fumigatus

From *Aspergillus fumigatus* culture on Sabouraud agar medium, one proceeds with the scraping of several culture plates and suspending of the scraped product in 30 ml of sterilised water.

The obtained suspension is stirred on a vortex, filtered through sterilised gauze and centrifuged at 15000 rpm for 10 minutes.

The supernatant is then eliminated and one proceeds to the freezing of the mycete pellet for 12 hours in liquid nitrogen.

The mycete pellet is then unfrozen and one proceeds with the homogenisation in a mortar with equal fungus/beads volume in a 50mM Tris-HCl buffer at a pH of about 7.5.

The obtained homogenate is centrifuged at 14000 rpm for 20 minutes and the protein content is evaluated.

The obtained hyphae can finally be directly used in the ELISPOT experiment according to the present invention at a concentration of 6-10 µg/ml. Alternatively, the hyphae are frozen at 20°C until the time of use, according to the present invention.

### CLINICAL STUDY

In order to evaluate if the count of the Aspergillus-specific T cells producing IFN-γ-TH1 and IL-10 (IL-10-TH2) through an enzymatic immunospot assay (ELISPOT) could improve the clinical diagnosis of IA, clinical tests were carried out on a series of 10 haematological patients with neutropenia and pulmonary infiltrations, three with proven IA infection and two healthy donors.

Peripheral blood mononuclear cells (PBMCs) were separate from each patient or healthy subject through Ficoll-Hypaque gradient centrifugation (Linaris Bettingen an Main, Germany) and were then spread on a 96-well polyvinylidene fluoride plate with either anti-gamma-IFN or IL-10 monoclonal antibodies (Mabtech, Nacka Strand, Sweden). The cells were tested with heat-inactivated conidia prepared from *Aspergillus fumigatus* isolated from a patient or with a watersoluble cell extract of *Aspergillus fumigatus* and with PHA, 1 x 10⁵ cells/well and 2.5x10⁴ cells/well on the aforesaid coated plates for gamma IFN and IL-10 assay, respectively, for sixteen hours.

All the test conditions were carried out in triplicate and the results were considered positive if the number of the spot forming cells (SFC)/106 cells in stimulated Aspergillus-antigen wells is twice that of the control wells (cells in non-stimulated Aspergillus-antigen wells) and there are at least 20 spot.

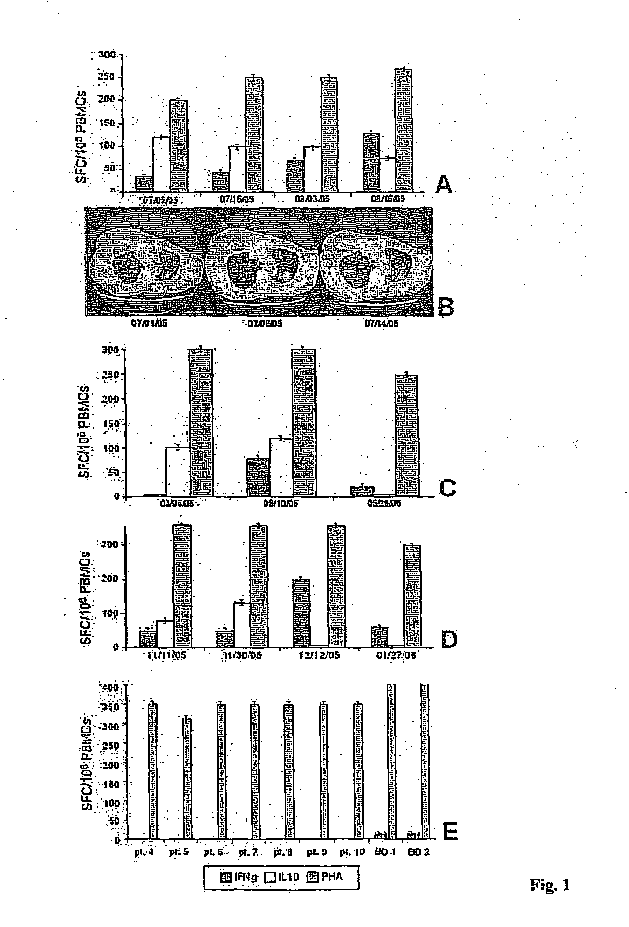

The first patient is a woman aged 59, affected with acute myeloid leukaemia (AML), who, during the neutropenic phase of the chemotherapeutic induction treatment (Fig. 1B), had a fever and a nodular lesion, surrounded by a ground glass attenuation halo, in the left lung, revealed by the high-resolution computed tomography (HRCT). The culture and molecular exams of the blood, urine, faeces and bronchoalveolar lavage fluid (BALf) repeatedly resulted negative for the presence of bacteria, fungi or virus. The galactomannan (GM) search was negative both on serum and BALf. The patient remained feverish notwithstanding the antibiotic and antimycotic therapy with vancomycin, meropenem and liposomal amphotericin (L-amB), at the dosage of 3 mg/kg/day. A second HRCT of the thorax revealed an enlargement of the nodular lesion and persistence of the ground glass attenuation halo. The blood GM remained negative (Fig. 1B). The L-amB dose was increased to 5 mg/kg/day. Several days later, the patient attained apyrexy, coinciding with the complete medullar recovery. After a week, HRCT showed a slight reduction both of the size of the pulmonary lesion and the surrounding halo (Fig. 1B). The patient was subjected to a resection of the bronchopneumonic focus via videothorascopy (VATS). The histological and immunohistochemical examinations of the operated section revealed a pulmonary Invasive Aspergillosis (IA).

The second patient is a 67-year-old woman who developed a fever and bilateral pulmonary infiltrations during the neutropenic phase following a chemotherapeutic induction cycle for AML. The culture and molecular examinations of the blood, urine, faeces and bronchoalveolar lavage fluid (BALf) were repeatedly negative for the presence of bacteria, fungi or viruses. With a second line, empirical antibiotic therapy, the resolution was obtained of all the pulmonary focuses except one, which showed enlarged on the HRCT. The culture and molecular examinations of the blood, urine, faeces and bronchoalveolar lavage fluid (BALf) remained negative for the presence of infection-responsible pathogen organisms. The galactomannan (GM) search was negative on blood, while it showed a positive result on BALf. Therapy with L-amB was undertaken at the dosage of 3 mg/kg/day. Upon the complete medullar healing, the patient had complete remission of the hemopathy. After a month of antifungal treatment, the pulmonary infiltrate was slightly reduced, presenting a small excavation sign. Before carrying out the consolidation chemotherapy, the patient underwent right lobe pulmonary surgical resection. The histological and immunohistochemical exams revealed a pulmonary IA. Several days after the surgical intervention, the patient had relapsed AML and died from the disease progression.

The third patient was a young man of 24 affected by acute lymphatic leukaemia (ALL), who developed a fever and a perihilar nodular legion of the left lung during the neutropenic phase of the induction chemotherapy. The culture and molecular exams of the blood, urine, faeces and bronchoalveolar lavage liquid (BALf) were repeatedly negative for the presence of bacteria, fungi or virus. The galactomannan (GM) search was negative both on serum and BALf. The cytological exam of BALf showed the presence of septate fungal hyphae. The antifungal therapy with voriconazole was undertaken at 200 mg intravenous doses twice a day. After two weeks of treatment, the nodular lesion size was unchanged and a small excavation area had appeared. Since the patient had had a haemoptysis episode, it was decided to surgically resection the upper segment of the left lower pulmonary lobe. The histological and immunohistochemical examinations of the operated section revealed a pulmonary IA. The patient attained complete remission of the hemopathy and completed two cycles of consolidation chemotherapy.

In patient 1, the ELISPOT method according to the present invention was carried out on peripheral blood samples collected between the first and second HRCT, at the same time as the third HRCT, and after one and three weeks from the execution of the VATS. The ELISPOT was positive for a response of Aspergillus-specific T cells, polarised type TH2 and producer of interleukin 10 (IL-10), in every determination, and showed a growing positive result for a response of Aspergillus-specific T cells, polarised type TH1 and producer of gamma-interferon (IFN-γ)(Fig. 1A)

In patient 2, the ELISPOT method according to the present invention was carried out at the time of the first HRCT, several days before the surgical operation and at the time of the AML relapse. The ELISPOT was positive for a response of Aspergillus-specific T cells, polarised type TH2 and producer of IL-10, at the first and second determination, and showed positive results for a response of Aspergillus-specific T cells, polarised type TH1 and producer of IFN-γ, at the second determination. The third determination was negative for any type of specific T-cell response (Fig. 1C).

In patient 3, the ELISPOT method was carried out at the same time as and fifteen days after the surgical operation, before the first and second consolidation chemotherapy cycle. The ELISPOT was positive for a response of Aspergillus-specific T cells, polarised type TH2 and producer of IL-10, at the first and second determination, and showed positive results for a response of Aspergillus-specific, polarised type TH1 and producer of IFN-γ at every determination (Fig. ID).

### Clinical results:

The method according to the present invention has therefore provided proof of the Invasive Aspergillosis in all three patients. In particular, the positive results of the ELISPOT according to the present invention are the only proof of the infection in patient 1, in which the diagnosis was otherwise attained only via surgical operation, all of the other methods resulting negative. The ELISPOT method according to the present invention then represents an effective improvement of the diagnostic power of the GM on BAL and of the BAL cytology in patients 2 and 3, respectively allowing a certain and not just suspected diagnosis.

Fig. E represents the results of ELISPOT in patients affected by pneumonia with non-fungal etiology, as negative controls.

## Claims

1. Method for the diagnosis of invasive aspergillosis which comprises carrying out an in vitro immunoenzymatic ELISPOT assay wherein the biological fluid drawn from the patient is placed in contact with an antigen of *Aspergillus jumigatus,* **characterized in that** said method comprises verifying the presence of IFN-γ producer T cells and IL-10 producer T cells and/or the ratio between IFN-γ producer T cells and IL-10 producer T cells.

2. Method according to claim 1, wherein said antigen is an extractive antigen of *Aspergillus fumigatus.*

3. Method according to claim 1, wherein said antigen is obtained from conidia of *Aspergillus, fumigatus.*

4. Method according to claim 1, wherein said antigen is obtained from hyphae of *Aspergillus fumigatus.*

5. Method according to claim 3, wherein said antigen is obtained through the following passages:
- incubation of the conidia for 3 days,
- collection of the conidia by means of washing of the fungal culture with sterilised water,
- filtration and subsequent centrifugation of the washing liquid,
- elimination of the supernatant,
- freezing and subsequent unfreezing of the conidia pellet,
- suspension in sterilised water,
- boiling and subsequent centrifugation,
- possible freezing.

6. Method according to claim 4, wherein said antigen is obtained through the following passages:
- collection of hyphae from culture plates though scraping and subsequent suspension in sterilised water,
- filtration and subsequent centrifugation,
- elimination of the supernatant,
- freezing and subsequent unfreezing of the mycete pellet,
- homogenisation of the mycete in the presence of a volume of inert material beads equal to the volume of the mycete,
- centrifugation,
- possible freezing.

7. Method according to claim 6, wherein said beads are made of glass.

8. Method according to claim 1, wherein said biological fluid is selected from among blood, bronchoalveolar lavage fluid or pleural fluid.

9. Method according to claim 1, wherein said biological fluid is selected from among all the mononuclear cells of the blood, of the bronchoalveolar lavage fluid, of the pleural fluid, or T lymphocytes of the blood, of the bronchoalveolar lavage fluid, of the pleural fluid.

10. Method according to claim 1, wherein the threshold value for IFN-γ or IL-10 is in the range of 15 - 25 SFCs.

11. Method according to claim 10, wherein said threshold value is 20 SFCs.

## Patentansprüche

1. Verfahren zur Diagnose von invasiver Aspergillose, welches die Durchführung eines immunoenzymatischen ELISPOT in vitro-Tests umfasse bei dem die dem Patienten entnommene Körperflüssigkeit in Kontakt mit einem Antigen von *Aspergillus fumigatus* gebracht wird, **dadurch gekennzeichnet, dass** das Verfahren eine Überprüfung des Vorhandenseins von IFN-γ herstellenden T-Zellen und IL-10 herstellenden T-Zellen und/oder eine Überprüfung des Verhältnisses zwischen IFN-γ herstellenden T-Zellen und IL-10 herstellenden T-Zellen umfasst.

2. Das Verfahren gemäß Anspruch 1, wobei das genannte Antigen ein extrahierbare Antigen von *Aspergillus fumigatus* ist.

3. Das Verfahren gemäß Anspruch 1, wobei das genannte Antigen aus Konidien von *Aspergillus fumigatus* gewonnen wird.

4. Das Verfahren gemäß Anspruch 1, wobei das genannte Antigen aus Hyphen von *Aspergillus fumigatus* gewonnen wird.

5. Das Verfahren gemäß Anspruch 3, wobei das genannte Antigen durch folgende Schritte gewonnen wird:
- Inkubation der Konidien für 3 Tage,
- Sammeln der Konidien durch Waschen der Pilzkultur mit sterilisiertem Wasser,
- Filtration und anschließende Zentrifugation der Waschflüssigkeit,
- Verwerfen des Überstands,
- Einfrieren und anschließendes Auftauen des Konidien-Pellets,
- Suspendieren in sterilisiertem Wasser,
- Aufkochen und anschließende Zentrifugation,
- Optional Einfrieren.

6. Das Verfahren gemäß Anspruch 4, wobei das genannte Antigen durch folgende Schritte gewonnen wird:
- Sammeln von Hyphen von Kulturplatten durch Ahschahen und anschließende Suspension in sterilisiertem Wasser,
- Filtration und anschließende Zentrifugation,
- Verwerfen des Überstands,
- Einfrieren und anschließendes Auftauen des Myceten-Pellets,
- Homogenisieren der Myceten in Gegenwart von Perlen aus inertem Material, deren Volumen gleich dem Volumen der Myceten ist,
- Zentrifugation,
- Optional Einfrieren.

7. Das Verfahren gemäß Anspruch 6, wobei die genannten Perlen aus Glas sind.

8. Das Verfahren gemäß Anspruch 1, wobei die genannte Körperflüssigkeit ausgewählt ist aus Blut, bronchoalveolärer Lavagetlüssigkeit oder pleuraler Flüssigkeit.

9. Das Verfahren gemäß Anspruch 1, wobei die genannte Körpernüssigkeit ausgewählt ist aus allen mononuklearen Zellen des Blutes, der bronchoalveolären Lavageflüssigkeit, der pleuralen Flüssigkeit, oder aus T-Lymphozyten des Blutes, der bronchoalveolären Lavageflüssigkeit, der pleuralen Flüssigkeit.

10. Das Verfahren gemäß Anspruch 1, wobei der Schwellenwert für IFN-γ oder IL-10 im Bereich von 15 - 25 SFCs liegt.

11. Das Verfahren gemäß Anspruch 10, wobei der genannte Schwellenwert 20 SFCs ist.

## Revendications

1. Méthode pour le diagnostic d'aspergillose invasive qui comprend la réalisation d'une analyse ELISPOT immunoenzymatique *in vitro* dans laquelle le fluide biologique prélevé au patient est mis en contact avec un antigène d'*Aspergillus fumigatus,* **caractérisée en ce que** ladite méthode comprend la vérification de la présence de cellules T productrices d'IFN-γ et de cellules T productrices d'IL-10 et/ou du rapport entre les cellules T productrices d'IFN-γ et les cellules T productrices d'IL-10.

2. Méthode selon la revendication 1, dans laquelle ledit antigène est un antigène extractif d'*Aspergillus fumigatus*.

3. Méthode selon la revendication 1, dans laquelle ledit antigène est obtenu à partir de conidie d'*Aspergillus fumigatus,*

4. Méthode selon la revendication 1, dans laquelle ledit antigène est obtenu à partir d'hyphes d'*Aspergillus fumigatus,*

5. Méthode selon la revendication 3, dans laquelle ledit antigène est obtenu par les étapes suivantes :
- Incubation de la conidie pendant 3 jours,
- Récupération de la conidie par lavage de la culture fongique avec de l'eau stérile,
- Filtration puis centrifugation du liquide de lavage,
- Elimination du surnageant,
- Congélation puis décongélation du culot de conidie,
- Suspension dans l'eau stérile,
- Ebullition puis centrifugation,
- Eventuellement congélation.

6. Méthode selon la revendication 4, dans laquelle ledit antigène est obtenu par les étapes suivantes :
- Récupération des hyphes à partir des plaques de culture par grattage puis suspension dans l'eau stérile,
- Filtration puis centrifugation,
- Elimination du surnageant,
- Congélation puis décongélation du culot de mycète,
- Homogénéisation du mycète en présence d'un volume de billes de matériau inerte égal au volume du mycète,
- Centrifugation,
- Eventuellement congélation.

7. Méthode selon la revendication 6, dans laquelle lesdites billes sont en verre.

8. Méthode selon la revendication 1, dans laquelle ledit fluide biologique est choisi parmi le sang, le fluide de lavage bronchoalvéolaire, ou le fluide pleural.

9. Méthode selon la revendication 1, dans laquelle ledit fluide biologique est choisi parmi toutes les cellules mononucléaires du sang, du fluide de lavage bronchoalvéolaire, du fluide pleural, ou les lymphocytes T du sang, du fluide de lavage bronchoalvéolaire, du fluide pleural.

10. Méthode selon la revendication 1, dans laquelle la valeur seuil pour l'IFN-γ ou l'IL-10 est dans la plage de 15-25 SFCs.

11. Méthode selon la revendication 10, dans laquelle ladite valeur seuil est 20 SFCs.
